# EUROPEAN PATENT SPECIFICATION

(11) **EP 2 982 986 B1**
(45) Date of publication and mention of the grant of the patent: **30.01.2019**
(21) Application number: 14779750.0
(22) Date of filing: 07.04.2014
(51) Int. Cl.: C12Q 1/6886

(54) **METHOD FOR MANUFACTURING GASTRIC CANCER PROGNOSIS PREDICTION MODEL**
VERFAHREN ZUR HERSTELLUNG EINES MODELLS ZUR PROGNOSE VON MAGENKREBS
PROCÉDÉ DE GÉNÉRATION D'UN MODÈLE DE PRÉDICTION DU PRONOSTIC D'UN CANCER GASTRIQUE

(30) Priority: 05.04.2013 KR 20130037336
(43) Date of publication of application: 10.02.2016
(73) Proprietor: Novomics Co., Ltd., Seoul (KR)
(72) Inventor: HUH, Yong-min, Seoul 137-930 (KR); NOH, Sung Hoon, Seoul 158-755 (KR); SUH, Jin Suck, Seoul 137-910 (KR); CHEONG, Jae-Ho, Seoul 135-280 (KR); PARK, Eun Sung, Seoul 138-891 (KR)
(74) Representative: Schiweck Weinzierl Koch Patentanwälte Partnerschaft mbB
(86) International application number: PCT/KR2014/002959
(87) International publication number: WO 2014/163445

(56) References cited:
- WO-A2-2012/081898
- KR-A- 20120 061 010
- US-A1- 2010 055 095
- JOANNA D HOLBROOK ET AL: "Deep sequencing of gastric carcinoma reveals somatic mutations relevant to personalized medicine", JOURNAL OF TRANSLATIONAL MEDICINE, BIOMED CENTRAL, LONDON, GB, vol. 9, no. 1, 25 July 2011 (2011-07-25), page 119, XP021105643, ISSN: 1479-5876, DOI: 10.1186/1479-5876-9-119
- STEFAN HEINDL ET AL: "Relevance of MET activation and genetic alterations ofand E-cadherin for cetuximab sensitivity of gastric cancer cell lines", JOURNAL OF CANCER RESEARCH AND CLINICAL ONCOLOGY, SPRINGER, BERLIN, DE, vol. 138, no. 5, 31 January 2012 (2012-01-31), pages 843-858, XP035043038, ISSN: 1432-1335, DOI: 10.1007/S00432-011-1128-4
- MOTOHISA TADA ET AL: "Prognostic significance of genetic alterations detected by high-density single nucleotide polymorphism array in gastric cancer", CANCER SCIENCE, vol. 101, no. 5, 1 May 2010 (2010-05-01), pages 1261-1269, XP055314934, JP ISSN: 1347-9032, DOI: 10.1111/j.1349-7006.2010.01500.x
- DREBBER U ET AL: "The overexpression of c-met as a prognostic indicator for gastric carcinoma compared to p53 and p21 nuclear accumulation", ONCOLOGY REPORTS, SPANDIDOS PUBLICATIONS, GR, vol. 19, no. 6, 1 June 2008 (2008-06-01), pages 1477-1483, XP002687318, ISSN: 1021-335X
- QUAYE, L. ET AL.: 'The Effects of Common Genetic Variants in Oncogenes on Ovarian Cancer Survival' CLINICAL CANCER RESEARCH vol. 14, no. 18, 2008, pages 5833 - 5839, XP055286157
- WINDER, T. ET AL.: 'Molecular Predictive and Prognostic Markers in Colon Cancer' CANCER TREATMENT REVIEWS vol. 36, no. 7, 2010, pages 550 - 556, XP027398554
- SIENA, S. ET AL.: 'Biomarkers Predicting Clinical Outcome of Epidermal Growth Factor Receptor-Targeted Therapy in Metastatic Colorectal Cancer' JOURNAL OF THE NATIONAL CANCER INSTITUTE vol. 101, no. 19, 2009, pages 1308 - 1324, XP008155321

## Description

### [Technical Field]

The present invention relates to a method of generating a novel prognosis prediction model through which it is possible to predict prognosis of gastric cancer through a genetic mutation comparative analysis method.

### [Background Art]

Gastric adeno-carcinoma is the second leading cause of death with 700,349 deaths in the year 2000 and the fourth most commonly diagnosed cancer in the world. It is considered a single heterogeneous disease with several epidemiologic and histo-pathologic characters. Treatment of gastric cancer is mainly based on clinical parameters like TNM (tumor, node, and metastasis) staging which decide whether patients should be treated by surgery alone or surgery plus chemotherapy. Unlike breast cancer and colon cancer, gastric cancer is clearly classified into stage 1 to stage 4 according to a TNM staging system. There is a great difference between stage 1 and stage 4, that is, a 5-year survival rate is equal to or greater than 90% in stage 1, and is equal to or less than 20% in stage 4. Therefore, it can be understood that the TNM staging system has very excellent prognosis predictability [Reference document, 7th edition of the AJCC cancer staging Manual: stomach. Ann Surg Oncol 2010; 17: 3077-3079]. Based on the staging system, gastric cancer can be generally divided into early gastric cancer, locally advanced gastric cancer, locally advanced invasive gastric cancer, metastatic gastric cancer and the like.

Even though surgery is the main treatment for operable gastric cancer, the recurrence rate is high in advanced cases. Multimodality treatment including chemotherapy and chemo-radiation has been introduced to prevent recurrence and to improve the prognosis of gastric cancer patients. However the optimal approach for individual patients is lacking as the clino-pathological heterogeneity of tumors and the different outcomes of patients in the same stage limit to predict responsibility of adjuvant chemotherapy even though these treatment options improved general clinical outcomes in patients.

Holbrook et al. (2011, Journal of Translational Medicine, vol. 9, no. 1, page 119) discussed the genetic characterization of 50 gastric adenocarcinoma samples and respective somatic mutations for establishing personalized medicine concerning gastric cancer.

Heindl et al. (2012, Journal of Cancer Research and Clinical Oncology, vol. 138, no. 5, pages 843-858) reported on the relevance of Met activation and genetic alterations of KRAS and E-cadherin for cetuximab sensitivity of gastric cancer cell lines.

WO 2012/081898 A2 describes markers for predicting stomach cancer prognosis, a composition and a kit for predicting stomach cancer prognosis, said composition and kit comprising preparations for determining an expression level of the marker, and to a method for predicting a stomach cancer prognosis using said marker.

Tada et al. (2010, Cancer Science, vol. 101, no. 5, pages 1261-1269) reported on prognostic significance of genetic alterations detected by high-density single nucleotide polymorphism array in gastric cancer.

Drebber et al. (2008, Oncology Reports, vol. 19, no. 6, pages 1477-1483) discussed the overexpression of c-met as a prognostic indicator for gastric carcinoma compared to p53 and p21 nuclear accumulation.

Despite the recent progress, challenges of cancer treatments for targeting therapeutic regimens specific to tumor types that differently onset and personalizing tumor treatments to ultimately maximize performance remain. Therefore, there is a need to perform experiments in which predictive information of patient responses according to various treatment choices is simultaneously provided.

### [Disclosure]

### [Technical Problem]

The present invention provides a method of generating a new prognosis prediction model based on a genetic mutation of gastric cancer patients.

### [Technical Solution]

In view of the above-described objects, the present invention provides a method of generating a prognosis prediction model for a subject diagnosed with gastric cancer. The method includes a step in which it is measured whether single nucleotide polymorphism is expressed in human KRAS, MET and PIK3CA genes in a biological sample including cancer cells collected from a subject; and a step in which a statistically significant set value range is determined according to an expression frequency of the single nucleotide polymorphism of the gene, and subjects are classified into a good prognosis group and a bad prognosis group for overall survival (OS) according to the set value range.

The prognosis prediction model may be used to predict clinical outcomes after surgical resection of all gastric cancers regardless of TNM staging.

### [Advantageous Effects]

The present invention provides a prognosis prediction model through which a statistically significant set value range according to an expression frequency of single nucleotide polymorphism of genesis set as a set value range for subjects of all gastric cancer patient groups regardless of TNM staging and the subjects can be classified into a good prognosis group and a bad prognosis group for overall survival. Therefore, it is possible to predict clinical outcomes after surgical resection of gastric cancer.

### [Description of Drawings]

FIG. 1 shows mRNA microarray results of tumor tissues and resulting group classification.
FIG. 2 shows Kaplan-Meier plots of groups.
FIGS. 3 to 5 show cross tabulation and chi-square analysis results of a group 2 and mutation relations.

### [Modes of the Invention]

Hereinafter, a configuration of the present invention will be described in detail.

The present invention provides a method of generating a prognosis prediction model for a subject diagnosed with gastric cancer. The method includes a step in which it is measured whether single nucleotide polymorphism in human KRAS, MET and PIK3CA genes is expressed in a high mRNA expression group and a low mRNA expression group; and a step in which a statistically significant set value range is determined by converting an expression frequency of the single nucleotide polymorphism of the gene into a percentage to obtain 3% or 7%, and subjects are classified into a good prognosis group and a bad prognosis group for overall survival (OS) according to the set value range.

According to the present invention, prognosis-related gene groups showing differential mRNA expression related to gastric cancer were selected and classified into a high mRNA expression group showing an increasing difference of 1.5 times or more and a low mRNA expression group showing a decreasing difference of 1.5 times or more based on a median value of mRNA expression on a biological sample including cancer cells collected from a subject. KRAS, MET and PIK3CA were selected as genes having a significantly high mutation probability in the classification group, and it was measured whether single nucleotide polymorphism was expressed in these genes in the high mRNA expression group and the low mRNA expression group. As a result, it was observed that, since an expression frequency of single nucleotide polymorphism of the genes was statistically significantly different between the high mRNA expression group and the low mRNA expression group, it can be applied as a classification reference that can be used to classify the good prognosis group and the bad prognosis group for overall survival.

Therefore, in the method of the present invention, according to the prognosis prediction model, subjects diagnosed with gastric cancer are classified into the good prognosis group and the bad prognosis group using an expression frequency of single nucleotide polymorphism of human KRAS, MET and PIK3CA genes as a classification reference. Such classification has a statistically significant value range.

The prognosis-related gene groups showing differential mRNA expression may undergo PCR or an array-based method.

Classification of the prognosis-related gene groups showing differential mRNA expression may be determined using various known statistical methods. Groups having a statistically significant set value, that is, a difference of 1.5 times or more can be classified into the high mRNA expression group and the low mRNA expression group.

Cancer is induced due to accumulation of genetic and epigenetic mutations in genomes. A change in one or two bases causes amino acid replacement, and thus an activity of proteins may be changed. Such single nucleotide polymorphism is associated with cancer diagnosis and prognosis. Therefore, in the present invention, an expression frequency of single nucleotide polymorphism of KRAS, MET and PIK3CA genes was applied to generate the prognosis prediction model.

Single nucleotide polymorphism of human KRAS is at least one selected from the group consisting of A146PT_g436ca, G10R_g28a, G12DAV_g35act, G12SRC_g34act, G13DAV_g38act, G13SRC_g37act, Q61EK_c181ga, Q61HHE_a183ctg and Q61LPR_a182tct. When a set value is determined, this is counted as one mutation and applied to the set value.

Here, an uppercase letter denotes a one-letter code of an amino acid, a lowercase letter denotes a base, and a number denotes a position of a base or an amino acid residue. For example, this means that g may be replaced with c or a at a gene position 436 in A146PT_g436ca, and amino acid alanine (A) may be replaced with proline (P) or threonine (T) at an amino acid position 146. Mutation examples of MET and PIK3CA given below also denote such meanings.

Single nucleotide polymorphism of the human MET is at least one selected from the group consisting of H1112L_a3335t, H1112Y_c3334t, M1268T_t3803c, N375S_a1124g, R988C_c2962t, T1010I_c3029t, Y1248HD_t3742cg and Y1253D_t3757g. When a set value is determined, this is counted as one single nucleotide polymorphism/ mutation and applied to the set value.

Single nucleotide polymorphism of the human PIK3CA is at least one selected from the group consisting of A1046V_c3137t, C420R_t1258c, E110K_g328a, E418K_g1252a, E453K_g1357a, E542KQ_g1624ac, E542VG_a1625tg, E545AGV_a1634cgt, E545D_g1635ct, E545KQ_g1633ac, F909L_c2727g, G1049R_g3145c, H1047RL_a3140gt, H1047RL_a3140gt, H1047Y_c3139t, H701P_a2102c, K111N_g333c, M1043I_g3129atc, M1043V_a3127g, N345K_t1035a, P539R_c1616g, Q60K_c178a, Q546EK_c1636ga, Q546LPR_a1637tcg, R88Q_g263a, S405F_c1214t, T1025SA_a3073tg, Y1021C_a3062g and Y1021HN_t3061ca. When a set value is determined, this is counted as one single nucleotide polymorphism/ mutation and applied to the set value.

A method of measuring the single nucleotide polymorphism is not specifically limited, and matrix-assisted laser desorption/ionization time-of-flight mass spectrometry (MALDI-TOF MS) may be used.

In the method of generating a prognosis prediction model according to the present invention, when the expression frequency of single nucleotide polymorphism is used as a reference for classifying the good prognosis group and the bad prognosis group, a statistically significant set value range is determined by converting the expression frequency of the single nucleotide polymorphism of the gene, and subjects can be classified into the good prognosis group and the bad prognosis group for overall survival based on the set value range.

The statistically significant set value range is determined by converting the expression frequency of the single nucleotide polymorphism of the gene into a percentage to obtain 3% or 7%, wherein when the expression frequency of single nucleotide polymorphism of human KRAS and PIK3CA genes is equal to or less than 3% and that of MET gene exceeds 7%, it can be classified into the bad prognosis group, and when the expression frequency of single nucleotide polymorphism of human KRAS and PIK3CA genes exceeds 3% and that of MET gene is equal to or less than 7%, it can be classified into the good prognosis group.

In the method of generating a prognosis prediction model according to the present invention, the good prognosis group has an increased likelihood of positive clinical outcome for a period of 5 years to 10 years for overall survival (OS) after surgical resection of all gastric cancers regardless of TNM staging and the bad prognosis group has a decreased likelihood of positive clinical outcomes for a period of 5 years to 10 years for overall survival (OS) after surgical resection of all gastric cancers regardless of TNM staging. The term "good prognosis" represents an increased likelihood of positive clinical outcomes, and the term "bad prognosis" represents a decreased likelihood of positive clinical outcomes as defined herein.

The prognosis prediction model according to the method of the present invention may be beneficial for predicting clinical outcomes after surgical resection of all gastric cancers regardless of TNM staging.

Unless otherwise defined, technical and scientific terms used herein have meanings that are generally understood by those skilled in the art. Terms will be defined below for the present invention.

The term "polynucleotide" refers in general to any polyribonucleotide or polydeoxyribonucleotide, for example, modified or non-modified RNA or DNA. In this specification, the term "polynucleotide" specifically includes cDNA.

The term "oligonucleotide" refers to a relatively short polynucleotide including a single-stranded deoxyribonucleotide, a single or double-stranded ribonucleotide, an RNA:DNA hybrid and double-stranded DNA without limitations. Oligonucleotides, for example, a single-stranded DNA probe oligonucleotide, are often synthesized by a chemical method in which, for example, a commercially available automated oligonucleotide synthesizer is used. However, the oligonucleotide may be prepared by various methods including an in vitro recombinant DNA-mediated technique and DNA expression in cells and organisms.

The term "differentially expressed gene" or "differential gene expression" refers to a gene that is activated at a higher or lower level in subjects with cancer such as gastric cancer than that in expression of normal or silent subjects. Also, genes activated at a higher or lower level in different stages of the same disease are included. The differentially expressed gene may be a gene that is activated or suppressed at a nucleic acid level or a protein level, or causes a different polypeptide product due to different splicing. Such a difference can be confirmed according to a change in, for example, an mRNA level of a polypeptide, surface expression, secretion or other distribution. In the present invention, when a difference between given gene expressions of normal subjects and subjects with a disease or various stages of subjects with a disease is 1.5 times or more "differential gene expression" is considered to be exhibited.

The term "normalized" related to a gene transcript or a gene expression product refers to a level of a transcript or a gene expression product with respect to an average level of a transcript/product of a reference gene set. Here, reference genes ("housekeeping genes") are selected based on a minimum variation thereof in patients, tissues or treatments, or reference genes are all tested genes. The latter case is referred to in general as "global normalization," and a relatively great number of tested genes in total is important, preferably, greater than 50. Specifically, the term "normalized" related to an RNA transcript refers to a transcription level with respect to an average of transcription levels of a reference gene set.

The terms "expression threshold value" and "defined expression threshold value" are interchangeably used and refer to a level of a gene or a gene product. At a level above the threshold value, the gene or the gene product is used as a predictive marker of a patient response. The threshold value is representatively and experimentally defined based on clinical studies. The expression threshold value may be selected as maximum sensitivity, maximum selectivity (for example, only responders of one drug should be selected), or a minimum error.

The term "gene amplification" refers to a process in which a plurality of replication products of genes or gene fragments is generated in specific cells or cell lines. A replicated region (elongation of amplified DNA) is often referred to as an "amplicon." Often, an amount of produced mRNA, that is, a degree of gene expression, also increases in proportion to the number of generated replication products of specific genes.

In this specification, the term "prognosis" is used to predict a likelihood of death from cancer or the progress (including recurrence, metastatic spread, and drug resistance) of neoplastic diseases such as gastric cancer herein. The term "prediction" is used herein to describe a likelihood of survival of a patient for a specific period without cancer recurrence after surgical resection of a major tumor. Such prediction may be clinically used to select a treatment method that is the most appropriate for any specific patient and determine the treatment method. Such prediction serves as a valuable indicator for predicting whether a patient is likely to beneficially respond to a therapeutic regimen, for example, a surgical procedure, or a patient is able to survive for a long time after completing surgery.

Unless otherwise indicated, the present invention may be performed using techniques of the related arts of molecular biology (including recombinant techniques), microbiology, cell biology and biochemistry.

### 1. Gene expression profiling

Gene expression profiling methods include a polynucleotide hybridization analysis-based method, a polynucleotide sequencing-based method, and a proteomics-based method. Exemplary methods of quantifying mRNA expression include northern blotting, in situ hybridization, an RNAse protection assay, and a PCR-based method such as a reverse transcription polymerase chain reaction (RT-PCR). Also, antibodies capable of recognizing two specific strands including two strands of DNA, two strands of RNA, two strands of a DNA-RNA hybrid or two strands of DNA-protein may be used. Representative sequencing-based gene expression analysis includes serial analysis of gene expression (SAGE) and gene expression analysis according to massively parallel signature sequencing (MPSS).

### 2. PCR-based gene expression profiling method

### a. Reverse transcriptase PCR (RT-PCR)

RT-PCR is one of the most sensitive and flexible quantitative PCR-based gene expression profiling methods, and may be used to compare mRNA levels in different sample groups such as normal tissues and tumor tissues with or without medication, characterize a gene expression pattern, determine closely related mRNA, and analyze RNA structures.

A first step includes isolation of mRNA from a target sample. mRNA may be extracted from, for example, frozen or archived paraffin-embedded and fixed (for example, formalin-fixed) tissue samples.

A general method of extracting mRNA is known in the art. A method of extracting RNA from paraffin-embedded tissues is disclosed in the documents ([Rupp and Locker, Lab Invest. 56: A67 (1987)], [De Andres et al., BioTechniques 18: 42044 (1995)]) and the like. In particular, RNA isolation may be performed using a purification kit, a buffer set and protease commercially available from, for example, Qiagen, according to the manufacturer's instructions. Other commercially available RNA isolation kits include MasterPure™ Complete DNA and RNA Purification Kit (EPICENTRE®, Madison, Wisconsin) and Paraffin Block RNA isolation kit (Ambion, Inc.). Pre-RNA may be isolated from the tissue sample using RNA Stat-60 (Tel-Test). RNA prepared from tumors may be isolated through, for example, cesium chloride density gradient centrifugation.

Since RNA is unable to be used as a template for PCR, the first step of gene expression profiling according to RT-PCR includes reverse transcription of an RNA template to cDNA, and then exponential amplification of a PCR reaction continues thereafter. In general, Avian Myeloblastosis Virus (AMV) Reverse Transcriptase (AMV-RT) and Moloney Murine Leukemia Virus Reverse Transcriptase (MMLV-RT) may be used as a reverse transcriptase. In a reverse transcription step, according to, representatively, an environment and a purpose of expression profiling, priming is performed using a specific primer, a random hexamer, or an oligo-dT primer. For example, extracted RNA may be reversely transcripted using GeneAmp RNA PCR kit (Perkin Elmer, California, USA) according to the manufacturer's instructions. Then, induced cDNA may be used as a template in a PCR reaction thereafter.

While various thermostable DNA-dependent DNA polymerases may be used in the PCR step, a Taq DNA polymerase is typically used. The Taq DNA polymerase has a 5'-3' nucleases activity, but has an insufficient low 3'-5' proofreading endonuclease activity. Therefore, in TaqMan® PCR, a 5'-nucleases activity of hybridizing a hybridization probe combined to a target amplicon of Taq or Tth polymerase is typically used, but any enzyme having a 5' nucleases activity may be used. Two oligonucleotide primers are used to generate a representative amplicon of the PCR reaction. A third oligonucleotide or a probe is designed to detect sequences of nucleotides positioned between two PCR primers. The probe has no stretchability due to a Taq DNA polymerase and is labeled with a reporter fluorescent dye and a quencher fluorescent dye. Any laser-induced emission from the reporter dye is removed by the quencher dye when two dyes are very close to each other, for example, when they are on the probe. During an amplification reaction, the Taq DNA polymerase cuts the probe using a template-dependent method. Generated probe fragments are dissociated in a solution, and a signal emitted from the reporter dye has no removing effect of a second fluorophore. One molecule of the reporter dye is emitted from each new synthesized molecule, and detection of an un-removed reporter dye serves as a reference for data quantitative analysis.

TaqMan® RT-PCR may be performed using commercially available devices, for example, ABI PRISM 7700TM Sequence Detection System™ (Perkin-Elmer-Applied Biosystems), or Lightcycler (Roche Molecular Biochemicals).

5'-nucleases assay data is initially represented as Ct, or a threshold value cycle. A fluorescent value is recorded during every cycle, and represents an amount of products amplified to a point due to the amplification reaction. A point at which a fluorescence signal is first recorded to be statistically significant is a threshold value cycle (Ct).

In order to minimize a variation effect between samples and an error, RT-PCR is generally performed using reference RNA (this is ideally expressed at a certain level between different tissues) and has no influence from an experimental treatment. RNA that is most frequently used to normalize a gene expression pattern is mRNA of housekeeping genes, glyceraldehyde-3-phosphate dehydrogenase (GAPD) and β-actin (ACTB).

Also, a new real time quantitative PCR is a technique for measuring PCR product accumulation through a dual labeled fluorescent probe (that is, a TaqMan® probe), and is commercially available with both a competitive quantitative PCR (here, an internal competitor of each target sequence is used for normalization) and a quantitative comparative PCR using normalization genes included in a sample or housekeeping genes of RT-PCR.

### b. MassARRAY system

In a mass array-based gene expression profiling method developed by Sequenom, Inc. cDNA obtained after RNA isolation and reverse transcription is spiked with a synthetic DNA molecule (competitor) (this matches a targeted cDNA region at all positions other than a single nucleotide), and is used as an internal standard. A cDNA/competitor mixture undergoes PCR amplification, a post-PCR shrimp alkaline phosphatase (SAP) treatment is performed and dephosphorylation of remaining nucleotides is caused. After an alkaline phosphatase is inactivated, PCR products from the competitor and cDNA undergo primer elongation, and these generate separate mass signals of competitor-and-cDNA-derived PCR products. After purification, these products are measured and distributed on a chip array in which components necessary for analysis using matrix-assisted laser desorption/ionization time-of-flight mass spectrometer (MALDI-TOF MS) analysis are already loaded. Then, cDNA present in the reaction is quantified by analyzing a peak area ratio in a generated mass spectrum.

### c. Other PCR-based methods

Other parallax displays; amplified fragment length polymorphism (iAFLP); BeadArray TM technique (Illumina, San Diego, California); commercially available Luminex 100 LabMAP system for a fast assay of gene expression and BeadsArray for Detection of Gene Expression (BADGE) using multicolor-coded microspheres (Luminex Corp.); and High-Coverage Expression Profiling (HiCEP) analysis.

### 3. Microarray

In fresh or paraffin-embedded tumor tissues, an expression profile of cancer-related genes may be measured. In this method, sequences of interest (including cDNA and oligonucleotides) are plated or arranged on a microchip substrate. Then, the arranged sequences are hybridized with specific DNA probes of cells or tissues of interest. Similarly to the RT-PCR method, a supply source of mRNA is the total RNA isolated typically from a human tumor or tumor cell lines, and corresponding normal tissues or cell lines. Therefore, RNA may be isolated from various major tumors or tumor cell lines. In a microarray technique, PCR amplified insertions of cDNA clones are provided on a substrate in a dense array manner. Preferably, 10,000 or more nucleotide sequences are applied to the substrate. Micro-arranged genes immobilized on a microchip with respect to 10,000 elements are appropriate for hybridization under strict conditions. Fluorescently labeled cDNA probes may be generated through reverse transcription of RNA extracted from tissues of interest and mixing of fluorescent nucleotides. The labeled cDNA probe applied to the chip is hybridized to have specificity to each spot of DNA on the array. In order to remove non-specifically bound probes, washing is completely performed, and then the chip is scanned by a confocal laser microscope or other detecting methods, for example, a CCD camera. When hybridization of the arranged elements is quantified, it is possible to evaluate excess of corresponding mRNA. When a dual-color fluorescent dye is used, separately labeled cDNA probes generated from two RNA supply sources are hybridized on the array for each pair. Therefore, relative excess of transcripts from two supply sources corresponding to each specified gene is simultaneously determined. Through hybridization in a small scale, convenient and rapid evaluation of expression patterns of a great number of genes is provided. Such a method has selectivity necessary for detecting rare transcripts (these are expressed in a small number of replication products for each cell) and performing detection with at least about twice a difference of a degree of expression in a reproducible manner. Microarray analysis may be performed using commercially available devices according to the manufacturer's protocol, for example, an Affymetrix GenChip technique or Incyte's microarray technique.

### 4. General descriptions of mRNA isolation, purification and amplification

A technique of profiling gene expression using paraffin-embedded tissues is described above. Based on a distinctive gene expression pattern identified in an observed tumor sample by analyzing finally obtained data, the best treatment choice(s) available for patients are determined.

An important object of the present invention is to provide prognosis information using measured expression of specific genes of gastric cancer tissues. In order to achieve such an object, it is necessary to compensate for (normalize) a difference in an amount of assayed RNA, a change in quality of used RNA, and other factors, for example, machine and worker differences. Therefore, in the assay, typically, a use of reference RNA including transcriptions from known housekeeping genes such as GAPD and ACTB is measured for mixing. Alternatively, normalization may be performed based on an average or a median signal (Ct) of assayed genes or a great number of all subsets thereof (global normalization approach). In the following example, a central standardization strategy was used and in order to perform normalization, subsets of screened genes selected based on a low correlation with clinical performance were used.

### [Examples]

Hereinafter, examples of the present invention will be described in detail. However, the following examples are only examples of the present invention, and the scope of the present invention is not limited to the following examples.

### <Example 1> Prognosis prediction subject selection and test design

A database of somatic cell mutations identified in various cancer types, which is possessed by Sanger Institute, was used. The present inventors developed an analysis method of detecting the presence of single nucleotide polymorphism (SNP) that is more generally generated in many different cancers.

For this purpose, based on 537 tumor tissue samples, 129 normal tissue samples, 125 FFPE tumor samples (gastric cancer patients who had undergone a gastrectomy as a primary treatment in Yonsei University Severance Hospital from 1999 to 2006) and 123 FFPE normal tissue samples, AKT1, BRAF, CTNNB1, FBWX7, GNAS, IDH1, JAK2, KIT, KRAS, MET, NRAS, PDGFRA, PDPK1, PHLPP2, PIK3CA and PIK3R1 were selected and 159 types of mutations shown therein were examined.

In order to detect single nucleotide polymorphism, a MALDI TOF MassArray system (Sequenom) was used. In this method, selected SNP near DNA was primarily amplified through PCR, a primer extension reaction was caused, and a potential SNP base was measured. Both a PCR primer and an extension primer were designed using Sequenom Assay Design software. This program enables a multi-reaction of different SNPs up to 29 per well. An initial PCR reaction was caused in a 384 well format according to the manufacturer's instructions, and EXO-SAP (Sequenom) was used to complete PCR. A primer extension reaction was caused using Sequenom's IPLEX chemistry and a protocol thereof. Then, in the IPLEX reaction, desalting was performed using Sequenom Clean Resin, and spotting on Spectrochip matrix chips was performed using Samsung Nanodispenser. Then, the chip was analyzed by Sequenom MassArray. Sequenom Typer Software interprets a generated mass spectrum and reports SNP based on the expected mass. All spectra generated are run in duplicate and are visually inspected.

Table 1 shows the results in which a mutation of one type or more generated in each gene was counted as 1, and calculated.

**[Table 1]**

| Classification | AK T1 | BR AF | CT NN B1 | FB WX 7 | GN AS | ID H1 | JA K2 | KIT | KR AS | ME T | NR AS | PD GF RA | PD PK 1 | PH LP P2 | PIK 3C A | PIK 3R1 |
|---|---|---|---|---|---|---|---|---|---|---|---|---|---|---|---|---|
| Tumor tissues-537 | 2 | 1 | 6 | 7 | 1 | 1 | 1 | 1 | 33 | 54 | 1 | 7 | 1 | 0 | 42 | 0 |
| Normal tissues-129 | 0 | 0 | 0 | 0 | 0 | 0 | 0 | 0 | 0 | 11 | 0 | 0 | 0 | 0 | 0 | 0 |
| FFPE-tumor sample-125 | 0 | 0 | 0 | 0 | 0 | 0 | 0 | 0 | 3 | 12 | 1 | 1 | 0 | 4 | 3 | 15 |
| FFPE-normal sample-123 | 0 | 0 | 0 | 0 | 0 | 0 | 0 | 0 | 1 | 12 | 0 | 1 | 0 | 4 | 0 | 12 |
| G.C cell lines-108 -4 repetition | 0 | 0 | 11 | 4 | 0 | 0 | 0 | 0 | 23 | 4 | 4 | 1 | 0 | 0 | 19 | 0 |
| Breast cancer cell lines-16-4 repetition | 0 | 11 | 0 | 0 | 0 | 0 | 0 | 0 | 11 | 0 | 0 | 0 | 0 | 0 | 0 | 0 |
| ETC | 0 | 0 | 0 | 0 | 0 | 0 | 0 | 4 | 1 | 6 | 0 | 2 | 0 | 0 | 1 | 0 |
| TOTAL - 1081 | 2 | 12 | 17 | 11 | 1 | 1 | 1 | 5 | 72 | 99 | 6 | 12 | 1 | 8 | 65 | 27 |

As shown in Table 1, it can be seen that a greater number of mutations of KRAS, MET, and PIK3CA were shown in tumors than normal tissues.

Mutation types according to single nucleotide polymorphism of KRAS, MET and PIK3CA are shown in Table 2.

**[Table 2]**

| Gene type | Mutation type |
|---|---|
| KRAS | A146PT_g436ca, G10R_g28a, G12DAV_g35act, G12SRC_g34act, G13DAV_g38act, G13SRC_g37act, Q61EK_c181ga, Q61HHE_a183ctg, Q61LPR_a182tct |
| MET | H1112L_a3335t, H1112Y_c3334t, M1268T_t3803c, N375S_a1124g, R988C_c2962t, T1010I_c3029t, Y1248HD_t3742cg, Y1253D_t3757g |
| PIK3CA | A1046V_c3137t, C420R_t1258c, E110K_g328a, E418K_g1252a, E453K_g1357a, E542KQ_g1624ac, E542VG_a1625tg, E545AGV_a1634cgt, E545D_g1635ct, E545KQ_g1633ac, F909L_c2727g, G1049R_g3145c, H1047RL_a3140gt, H1047RL_a3140gt, H1047Y_c3139t, H701P_a2102c, K111N_g333c, M1043I_g3129atc, M1043V_a3127g, N345K_t1035a, P539R_c1616g, Q60K_c178a, Q546EK_c1636ga, Q546LPR_a1637tcg, R88Q_g263a, S405F_c1214t, T1025SA_a3073tg, Y1021C_a3062g, Y1021HN_t3061ca |
| An uppercase letter denotes a one-letter code of an amino acid, a number between uppercase letters denotes a position of an amino acid, the left number denotes an amino acid before replacement, and the right number denotes an amino acid after replacement. A lowercase letter denotes a base, a number between lowercase letters denotes a position of a base in a gene, the left number denotes a base before replacement, and the right number denotes a base after replacement. | |

Based on the results, chi-square analysis was performed for 545 tumor tissue samples and 129 normal tissue samples, possessed by Yonsei University Severance Hospital. As a result, p-values of KRAS, MET and PIK3CA were 0.001 or less. That is, it can be understood that normal tissues and tumor tissues have a clear distinction regarding whether there is a mutation.

In the samples, 350 tumor tissues were analyzed by mRNA microarray and it was examined whether there was a mutation related to the corresponding prognosis in mRNA groups. For this purpose, 350 samples were analyzed by mRNA unsupervised clustering, divided into groups, and a Kaplan-Meier Plot and log rank test were performed thereon, and thus prognosis-related groups were confirmed. That is, 350 samples were extracted as original raw data, quartile normalization was performed thereon, conversion into a log base 2 was performed, and then median centering was performed. Prognosis-related genes were grouped through a dispersive filtering method. Finally, 15 probes showing an increase or a decrease of 1.5 times or more than a median value were used to extract 5432 genes, and cluster analysis was performed using Cluster and Treeview

### (http://rana.lbl.gov/EigenSoftware.htm).

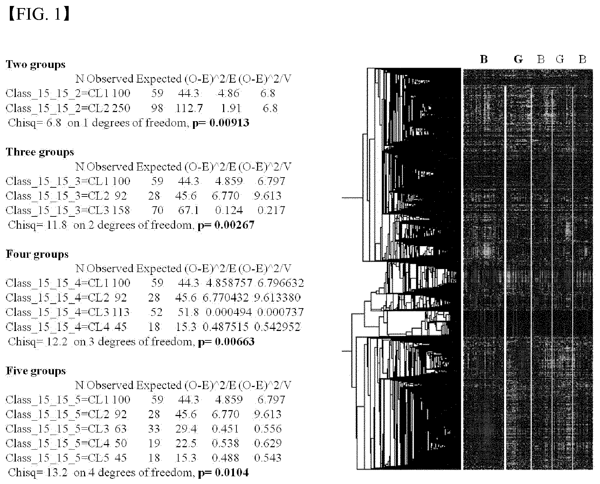

In the groups, samples having KRAS, MET and PIK3CA mutations were arranged. Through a chi-square analysis technique, a correlation with the corresponding mutation between the groups was examined. The mRNA microarray results of tumor tissues are shown in FIG. 1.

FIG. 1 and Table 3 show p-values when samples were classified into two groups (CL1 and CL2), when samples were classified into three groups (CL1, CL2, and CL3), when samples were classified into four groups (CL1, CL2, CL3, and CL4), and when samples were classified into five groups (CL1, CL2, CL3, CL4, and CL5). For example, a group 2 was classified into two groups (CL1 and CL2) and it is possible to know whether there is a mutation of KRAS, MET and PIK3CA in each of the groups.

**[Table 3]**

| CHI-SQUARE | KRAS | MET | PIK3CA |
|---|---|---|---|
| Group 2 | 0.046 | 0.064 | 0.015 |
| Group 3 | 0.003 | 0.180 | 0.002 |
| Group 4 | 0.008 | 0.314 | 0.005 |
| Group 5 | 0.016 | 0.181 | 0.007 |

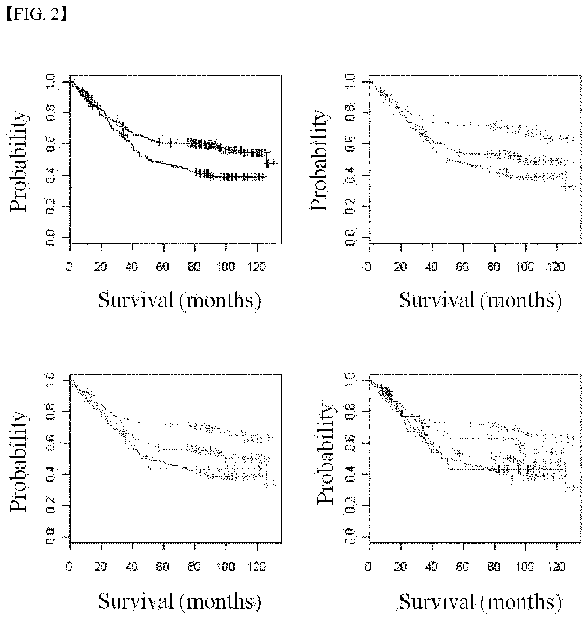

FIG. 2 shows Kaplan-Meier plots of groups.

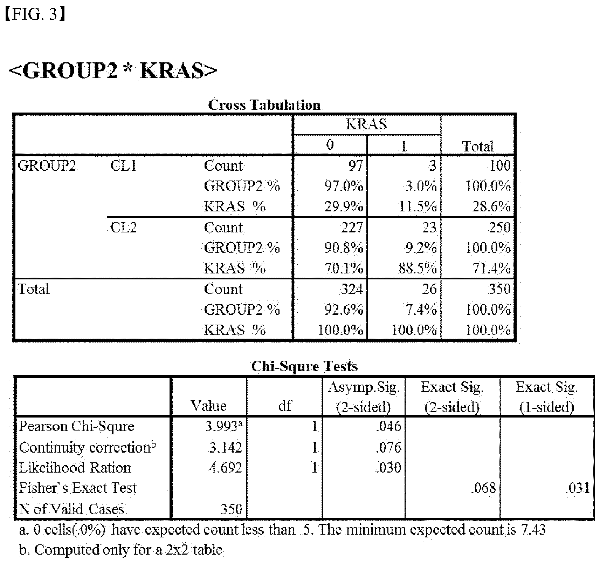

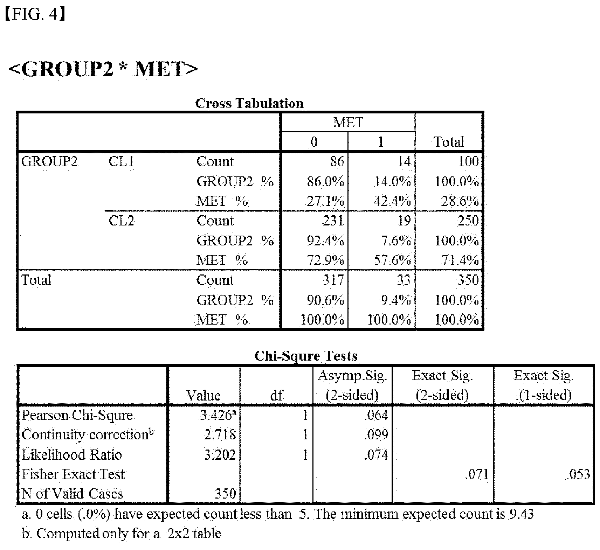

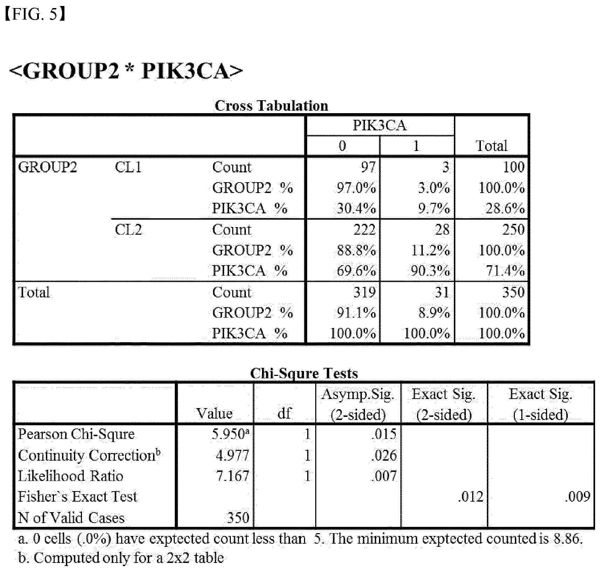

FIGS. 3 to 5 show cross tabulation and chi-square analysis results of the group 2 and mutation relations.

As shown in FIGS. 3 to 5, a mutation frequency of KRAS and PIK3CA genes was high in a good prognosis class 2, and a mutation frequency of KRAS and PIK3CA genes was low in a bad prognosis class 1. Also, a mutation frequency of MET genes was low in the good prognosis class 2, and a mutation frequency of MET genes was high in the bad prognosis class 1.

Further is described that the present invention can be used as a diagnostic kit in the field of recurrence prognosis prediction of gastric cancer.

## Claims

1. A method of generating a prognosis prediction model for a subject diagnosed with gastric cancer, the method comprising:
selecting prognosis-related gene groups showing differential mRNA expression related to gastric cancer and classifying into the high mRNA expression group showing an increasing difference of 1.5 times or more and the low mRNA expression group showing a decreasing difference of 1.5 times or more based on a median value of mRNA expression in a biological sample including cancer cells collected from a subject, and then measuring whether single nucleotide polymorphism in human KRAS, MET and PIK3CA genes is expressed in the high mRNA expression group and the low mRNA expression group; and
determining a statistically significant set value range by converting an expression frequency of the single nucleotide polymorphism of the gene into a percentage to obtain 3% or 7% and classifying into a good prognosis group and a bad prognosis group for overall survival (OS) according to the set value range,
wherein, when the expression frequency of single nucleotide polymorphism of human KRAS and PIK3CA genes exceeds 3% and that of MET gene is equal to or less than 7%, it can be classified into the good prognosis group, and
when the expression frequency of single nucleotide polymorphism of human KRAS and PIK3CA genes is equal to or less than 3% and that of MET gene exceeds 7%, it can be classified into the bad prognosis group,
wherein, the good prognosis group has an increased likelihood of positive clinical outcomes for a period of 5 years to 10 years for overall survival (OS) after surgical resection of all gastric cancers regardless of TNM staging, and the bad prognosis group has a decreased likelihood of positive clinical outcomes for a period of 5 years to 10 years for overall survival (OS) after surgical resection of all gastric cancers regardless of TNM staging, and
the single nucleotide polymorphism of human KRAS is at least one selected from the group consisting of A146PT_g436ca, G10R_g28a, G12DAV_g35act, G12SRC_g34act, G13DAV_g38act, G13SRC_g37act, Q61EK_c181ga, Q61HHE_a183ctg and Q61LPR_a182tct;
the single nucleotide polymorphism of human MET is at least one selected from the group consisting of H1112L_a3335t, H1112Y_c3334t, M1268T_t3803c, N375S_a1124g, R988C_c2962t, T1010I_c3029t, Y1248HD_t3742cg and Y1253D_t3757g; and
the single nucleotide polymorphism of human PIK3CA is at least one selected from the group consisting of A1046V_c3137t, C420R_t1258c, E110K_g328a, E418K_g1252a, E453K_g1357a, E542KQ_g1624ac, F542VG_a1625tg, F545AGV_a1634cgt, E545D_g1635ct, E545KQ_g1633ac, F909L_c2727g, G1049R_g3145c, H1047RL_a3140gt, H1047RL_a3140gt, H1047Y_c3139t, H701P_a2102c, K111N_g333c, M1043I_g3129atc, M1043V_a3127g, N345K_t1035a, P539R_c1616g, Q60K_c178a, Q546EK_c1636ga, Q546LPR_a1637tcg, R88Q_g263a, S405F_c1214t, T1025SA_a3073tg, Y1021C_a3062g and YI021HN_t3061ca,
wherein, an uppercase letter denotes a one-letter code of an amino acid, a lowercase letter denotes a base, and a number denotes a position of a base or an amino acid residue.

## Patentansprüche

1. Verfahren zur Erstellung eines Vorhersage-Modells zur Prognose für einen Patienten, bei dem Magenkrebs diagnostiziert wurde, wobei das Verfahren Folgendes umfasst:
Auswählen von mit der Prognose verbundenen Gengruppen, die eine differentielle mRNA-Expression in Bezug auf Magenkrebs zeigen, und Klassifizieren in die Gruppe mit hoher mRNA-Expression, die eine zunehmende Differenz von 1,5 Mal oder mehr zeigt, und in die Gruppe mit niedriger mRNA-Expression, die eine abnehmende Differenz von 1,5 Mal oder mehr zeigt, basierend auf einem Mittelwert der mRNA-Expression in einer biologischen Probe, einschließlich Krebszellen, die von einem Patienten gewonnen wurden, und anschließendes Messen, ob der Einzelnukleotid-Polymorphismus in menschlichen KRAS-, MET- und PIK3CA-Genen in der Gruppe mit hoher mRNA-Expression und in der Gruppe mit niedriger mRNA-Expression exprimiert ist; und
Bestimmen eines statistisch signifikanten Sollwertbereichs durch Umwandeln einer Expressionsfrequenz des Einzelnukleotid-Polymorphismus des Gens in einen Prozentsatz, um 3% oder 7% zu erhalten, und Klassifizieren in eine gute Prognosegruppe und eine schlechte Prognosegruppe für das Gesamtüberleben (OS) nach dem Sollwertbereich,
wobei, wenn die Expressionsfrequenz des Einzelnukleotid-Polymorphismus der menschlichen KRAS- und PIK3CA-Gene 3% überschreitet und die des MET-Gens gleich oder weniger als 7% beträgt, er in die gute Prognosegruppe klassifiziert werden kann, und
wenn die Expressionsfrequenz des Einzelnukleotid-Polymorphismus der menschlichen KRAS- und PIK3CA-Gene gleich oder weniger als 3% beträgt, und die des MET-Gens 7% überschreitet, er in die schlechte Prognosegruppe klassifiziert werden kann,
wobei die gute Prognosegruppe eine erhöhte Wahrscheinlichkeit von positiven klinischen Ergebnissen für einen Zeitraum von 5 Jahren bis 10 Jahren für das Gesamtüberleben (OS) nach chirurgischer Resektion aller Magenkarzinome, unabhängig von TNM-Staging, hat und die schlechte Prognosegruppe eine verminderte Wahrscheinlichkeit von positiven klinischen Ergebnissen für einen Zeitraum von 5 Jahren bis 10 Jahren für das Gesamtüberleben (OS) nach chirurgischer Resektion aller Magenkarzinome, unabhängig von TNM-Staging, hat und
der Einzelnukleotid-Polymorphismus des menschlichen KRAS wenigstens einer ist, ausgewählt aus der Gruppe, bestehend aus A146PT_g436ca, G10R_g28a, G12DAV_g35act, G12SRC_g34act, G13DAV_g38act, G13SRC_g37act, Q61EK_c181ga, Q61HHE_a183ctg und Q61LPR_a182tct;
der Einzelnukleotid-Polymorphismus des menschlichen MET wenigstens einer ist, ausgewählt aus der Gruppe, bestehend aus H1112L_a3335t, H1112Y_c3334t, M1268T_t3803c, N375S_a1124g, R988C_c2962t, T10101_c3029t, Y1248HD_t3742cg und Y1253D_t3757g; und
der Einzelnukleotid-Polymorphismus des menschlichen PIK3CA wenigstens einer ist, ausgewählt aus der Gruppe, bestehend aus A1046V_c3137t, C420R_t1258c, E110K_g328a, E418K_g1252a, E453K_g1357a, E542KQ_g1624ac, E542VG_a1625tg, E545AGV_a1634cgt, E545D_g1635ct, E545KQ_g1633ac, F909L_c2727g, G1049R_g3145c, H1047RL_a3140gt, H1047RL_a3140gt, H1047Y_c3139t, H701P_a2102c, K111N_g333c, M1043I_g3129atc, M1043V_a3127g, N345K_t1035a, P539R_c1616g, Q60K_c178a, Q546EK_c1636ga, Q546LPR_a1637tcg, R88Q_g263a, S405F_c1214t, T1025SA_a3073tg, Y1021C_a3062g und Y1021HN_t3061ca,
wobei ein Großbuchstabe einen Ein-Buchstaben-Code einer Aminosäure bezeichnet, ein Kleinbuchstabe eine Base bezeichnet und eine Zahl eine Position einer Base oder eines Aminosäurerestes bezeichnet.

## Revendications

1. Procédé de génération d'un modèle de prédiction du pronostic pour un sujet souffrant de cancer gastrique diagnostiqué, le procédé comportant:
sélectionner des groupes de gènes liés au pronostic montrant une expression différentielle d'ARNm associée au cancer gastrique et classifier dans le groupe de l'expression d'ARNm élevée montrant une différence croissante de 1.5 fois ou plus, et le groupe de l'expression d'ARNm faible montrant une différence réduite de 1.5 fois ou plus basé sur une valeur moyenne de l'expression d'ARNm dans un échantillon biologique y compris des cellules cancéreuses prélevées sur un sujet, et ensuite mesurer si un polymorphisme nucléotidique simple dans des gènes humains KRAS, MET et PIK3CA est exprimé dans le groupe de l'expression d'ARNm élevée et le groupe de l'expression d'ARNm faible, et
déterminer une plage de valeurs de consigne statistiquement pertinente en convertissant une fréquence d'expressions du polymorphisme nucléotidique simple du gène dans un pourcentage pour obtenir 3% ou 7% et classifier dans un groupe de bon pronostic et un groupe de mauvais pronostic pour la survie globale (OS) selon la plage de valeurs de consigne,
dans laquelle, lorsque la fréquence d'expressions du polymorphisme nucléotidique simple des gènes humains KRAS et PIK3CA excède 3%, et celle du gène MET est égal à ou moins de 7%, elle peut être classifiée dans le groupe de bon pronostic, et
lorsque la fréquence d'expressions du polymorphisme nucléotidique simple des gènes humains KRAS et PIK3CA est égal à ou moins de 3% et celle du gène MET excède 7%, elle peut être classifiée dans le groupe de mauvais pronostic,
le groupe de bon pronostic ayant une probabilité élevée de résultats cliniques positifs pour une période de 5 années à 10 années pour la survie globale (OS) après résection chirurgicale de tous cancers gastriques indépendamment de la classification TNM, et le groupe de mauvais pronostic ayant une probabilité réduite des résultats cliniques positifs pour une période de 5 années à 10 années pour la survie globale (OS) après résection chirurgicale de tous cancers gastriques indépendamment de la classification TNM, et
le polymorphisme nucléotidique simple de KRAS humain étant au moins un choisi parmi le groupe constitué de A146PT_g436ca, G10R_g28a, G12DAV_g35act, G12SRC_g34act, G13DAV_g38act, G13SRC_g37act, Q61EK_c181ga, Q61HHE_a183ctg et Q61LPR_a182tct;
le polymorphisme nucléotidique simple de MET humain étant au moins un choisi parmi le groupe constitué de H1112_a3335t, H1112Y_c3334t, M1268T_t3803c, N375S_a1124g, R988C_c2962t, T1010I_c3029t, Y1248HD_t3742cg et Y1253D_t3757g; et
le polymorphisme nucléotidique simple de PIK3CA humain étant au moins un choisi parmi le groupe constitué de A1046V_c3137t, C420R_t1258c, E110K_g328a, E418K_g1252a, E453K_g1357a, E542KQ_g1624ac, E542VG_a1625tg, E545AGV_a1634cgt, E545D_g1635ct, E545KQ_g1633ac, F909L_c2727g, G1049R_g3145c, H1047RL_a3140gt, H1047RL_a3140gt, H1047Y_c3139t, H701P_a2102c, K111N_g333c, M1043I_g3129atc, M1043V_a3127g, N345K_t1035a, P539R_c1616g, Q60K_c178a, Q546EK_c1636ga, Q546LPR_a1637tcg, R88Q_g263a, S405F_c1214t, T1025SA_a3073tg, Y1021C_a3062g et Y1021HN_t3061ca,
une lettre majuscule désignant un code à une lettre d'un acide aminé, une lettre minuscule désignant une base, et un nombre désignant une position d'une base ou un résidu d'acide aminé.
